# FASCICULE DE BREVET EUROPEEN

(11) **EP 1 753 858 B1**
(45) Date de publication et mention de la délivrance du brevet: **03.03.2010**
(21) Numéro de dépôt: 05773198.6
(22) Date de dépôt: 25.05.2005
(51) Int. Cl.: C12N 1/20, C12P 17/02, A61K 31/357, A61P 31/04, A61P 35/00, A61P 31/12

(54) **NOUVELLE SOUCHE SACCHAROTHRIX ET SES ANTIBIOTIQUES DERIVES, NOTAMMENT MUTACTIMYCINES ET ALDGAMYCINES**
NEUER SACCHAROTRIXSTAMM UND DAVON ABGELEITETE ANTIBIOTIKA, INSBESONDERE MUTACTIMYCINE UND ALDGAMYCINE
NOVEL SACCHAROTHRIX STRAIN AND ANTIBIOTICS DERIVED THEREFROM, I.E. MUTACTIMYCINS AND ALDGAMYCINS

(30) Priorité: 26.05.2004 FR 0405659
(43) Date de publication de la demande: 21.02.2007
(73) Titulaire: Institute National Polytechnique de Toulouse, 31000 Toulouse (FR)
(72) Inventeur: ZITOUNI, Abdelghani, 16 300 (Alger) (DZ); SABOU, Nasserdine, 16 300 (Alger) (DZ); MATHIEU, Florence, F-31 750 Escalquens (FR); LEBRIHI, Ahmed, F-31 450 Noueilles (FR)
(74) Mandataire: Warcoin, Jacques
(86) Numéro de dépôt international: PCT/FR2005/001291
(87) Numéro de publication internationale: WO 2005/118777

(56) Documents cités:
- EP-A- 0 257 615
- MIZOBUCHI S ET AL: "ALDGAMYCIN G A NEW MACROLIDE ANTIBIOTIC" JOURNAL OF ANTIBIOTICS (TOKYO), vol. 39, no. 12, 1986, pages 1776-1778, XP009041231 ISSN: 0021-8820
- ZITOUNI A ET AL: "MUTACTIMYCIN PR, A NEW ANTHRACYCLINE ANTIBIOTIC FROM SACCHARONTHRIX SP. SA 103 II. PHYSICO-CHEMICAL PROPERTIES AND STRUCTURE ELUCIDATION" JOURNAL OF ANTIBIOTICS, JAPAN ANTIBIOTICS RESEARCH ASSOCIATION, TOKYO, JP, vol. 57, no. 6, juin 2004 (2004-06), pages 373-378, XP009041232 ISSN: 0021-8820
- ZITOUNI A ET AL: "MUTACTIMYCIN PR, A NEW ANTHRACYCLINE ANTIBIOTIC FROM SACCHAROTHRIX SP. SA 103 I. TAXONOMY, FERMENTATION, ISOLATION AND BIOLOGICAL ACTIVITIES" JOURNAL OF ANTIBIOTICS, JAPAN ANTIBIOTICS RESEARCH ASSOCIATION, TOKYO, JP, vol. 57, no. 6, juin 2004 (2004-06), pages 367-372, XP009041233 ISSN: 0021-8820
- SPEITLING M ET AL: "DEMETHYL MUTACTIMYCINS, NEW ANTHRACYCLINE ANTIBIOTICS FROM NOCARDIA AND STREPTOMYCES STRAINS" JOURNAL OF ANTIBIOTICS, JAPAN ANTIBIOTICS RESEARCH ASSOCIATION, TOKYO, JP, vol. 51, no. 8, août 1998 (1998-08), pages 693-698, XP009041230 ISSN: 0021-8820
- MIKAMI Y ET AL: "SO-75R1, A NEW MUTACTIMYCIN DERIVATIVE PRODUCED BY NOCARDIA BRASILIENSIS" JOURNAL OF ANTIBIOTICS, JAPAN ANTIBIOTICS RESEARCH ASSOCIATION, TOKYO, JP, vol. 45, no. 6, juin 1992 (1992-06), pages 995-997, XP009041246 ISSN: 0021-8820
- GRUND E ET AL: "TRANSFER OF FIVE NOCARDIOPSIS SPECIES TO THE GENUS SACCHAROTHRIX LABEDA ET AL. 1984" SYSTEMATIC AND APPLIED MICROBIOLOGY, vol. 12, no. 3, 1989, pages 267-274, XP009041374 ISSN: 0723-2020
- LABEDA D P ET AL: "SACCHAROTHRIX NEW-GENUS OF THE ACTINOMYCETALES RELATED TO NOCARDIOPSIS" INTERNATIONAL JOURNAL OF SYSTEMATIC BACTERIOLOGY, vol. 34, no. 4, 1984, pages 426-431, XP009041167 ISSN: 0020-7713
- SABAOU NASSERDINE ET AL: "Les sols du oasis du Sahara algérien, source d'actinomycètes, rares producteurs d'antibiotiques" SECHERESSE (MONTROUGE), vol. 9, no. 2, juin 1998 (1998-06), pages 147-153, XP009041373 ISSN: 1147-7806

## Description

L'invention a pour objet une nouvelle souche actinomycète *Saccharothrix* SA 103 déposée à la CNCM le 6 février 2004 sous le numéro 1-3160 ou une souche mutante de celle-ci, un procédé de production d'un bouillon de culture, d'un concentrat actif et de composés actifs à partir d'une culture de cette souche SA 103.

Les agents antimicrobiens sont largement utilisés dans de nombreux domaines : santé humaine, vétérinaire, phytopathologie, industrie alimentaire, traitement du cuir et du bois, etc. La plupart de ces agents sont produits par des microorganismes, dont les actinomycètes sont parmi les plus importants.

On cherche toujours de nouveaux agents antimicrobiens, en particulier des antibiotiques antibactériens.

En effet, on sait que de par la nature même des bactéries, celles-ci prolifèrent de manière exponentielle, et augmentent ainsi leurs chances de devenir résistantes à des antibiotiques, ceci suite à des mutations génétiques.

L'utilisation inadéquate d'antibiotiques dans le domaine animal, notamment l'homme, ou végétal, mais aussi dans l'industrie alimentaire ou l'industrie du cuir, accroît la capacité mutationnelle des bactéries, et donc leur résistance à des antibiotiques.

Une solution à ce problème est bien sûr d'administrer à bon escient les antibiotiques, mais cette solution ne peut être que préventive.

Il existe donc un besion, dans l'état actuel de la technique, de développer de nouveaux antibiotiques. Pour ce faire, les recherches se sont portées sur les microorganismes extrêmophiles.

Les inventeurs ont isolé une nouvelle souche actinomycète *Saccharothrix* SA 103 présente dans le sol sud saharien de l'Algérie, et l'inventeur Ahmed LEBRIHI, Le Paraillet, 31450 NOUEILLES) l'a déposée à la Collection Nationale de Cultures de Microorgansimes (CNCM) le 16 février 2004 sous le numéro I-3160.

Ils ont également découvert que cette souche produisait des composés actifs de la classe des anthracyclines, dont certains n'ont jamais été identifiés auparavant, notamment des mutactimycines (PR, F, G), mais également des composés actifs de la classe des anthracyclines, notamment des aldgamycines (G, H et P10b). Ces composés actifs présentent une activité antibactérienne, en particulier contre les bactéries Gram-positives, mais également une activité antivirale, antiproliférative et anticancéreuse.

Ils ont dès lors mis au point un procédé d'obtention de ces composés à partir de la souche *Saccharothrix* SA 103 déposée à la CNCM le 16 février 2004 sous le numéro I-3160, lequel procédé présente l'avantage d'être simple à mettre en oeuvre.

Ceci est justement l'objet de la présente invention.

Ainsi, l'invention a tout d'abord pour objet la souche actinomycète *Saccharothrix* SA 103 déposée à la CNCM le 16 février 2004 sous le numéro I-3160 ou une souche mutante de celle-ci.

Le document Sabaou et al., Sécheresse (Montrouge), Viol. 9, no. 2. juin 1998, pages 147-153, fait référence à des souches du genre *Saccharothrix* isolées du sol sud saharien de l'Algérie et à l'importance de ces souches pour la production de nouveaux antibiotiques. Toutefois, il ne décrit pas ni ne suggère la souche actinomycète *Saccharothrix* SA 103 telle que définie ci-après.

Par « souche mutante », « mutant », « souche variante » ou « variant », on entend désigner de manière indifférente dans la présente demande une souche de *Saccharothrix* pouvant être obtenue par mutation sélective à partir de la souche *Saccharothrix* SA 103 en conservant la capacité de produire au moins l'un des composés actifs décrits ci-après. Les techniques de mutation sont connues de l'homme du métier et consistent à mettre la souche *Saccharothrix* SA 103 en présence d'un agent mutagène physique, tel qu'un rayonnement, ou d'un agent mutagène chimique, par exemple l'acriflavine, puis à sélectionner dans un milieu approprié les mutants d'intérêt restants en utilisant leur spectre antiobiotique (méthode d'inhibition de microorganismes telle que par exemple la méthode de la concentration minimale inhibitrice ou CMI, etc...).

Les caractéristiques de la souche *Saccharothrix* SA 103 selon l'invention sont décrites dans la partie « Exemples » de la présente demande.

La souche *Saccharothrix* SA 103 selon l'invention ou l'un de ses mutants est mise en culture dans un milieu contenant une variété de substances nutritives généralement utilisées pour la croissance des actinomycètes. Par exemple, comme source de carbone, on peut utiliser du glucose, de la glycérine, du sucrose, de l'amidon, du maltose, ou bien des huiles animales ou végétales. Comme source d'azote on peut utiliser, par exemple, de l'azote organique tel que de la farine de soja, des extraits de viande, un extrait de levure, une peptone, de l'eau de macération du maïs, du tourteau de coton ou de la farine de poisson. On peut utiliser de l'azote inorganique tel que par exemple du sulfate d'ammonium, du chlorure d'ammonium, du nitrate de sodium ou du phosphate d'ammonium. Si cela est nécessaire, on peut encore ajouter du chlorure de sodium, du chlorure de potassium, du phosphate de potassium ou des sels métalliques bivalents tels que Mg⁺⁺, Ca⁺⁺, Zn⁺⁺, Fe⁺⁺, Cu⁺⁺, Mn⁺⁺ ou Ni⁺⁺, et des acides aminés ou des vitamines. On peut utiliser des géloses inclinées.

La souche SA 103 selon l'invention que les inventeurs ont réussi à isoler est une souche extrêmement rare. En effet, une seule colonie a été mise en évidence sur plus de 120 échantillons analysés avec ou sans produits sélectifs tels que par exemple les antibiotiques cités précédemment.

Un autre objet de la présente invention est un procédé de production d'un bouillon de culture à partir d'une culture de la souche actinomycète *Saccharothrix* SA 103 et/ou d'au moins l'une de ses souches mutantes selon l'invention, **caractérisé en ce qu**'il comprend les étapes suivantes :
a) fermentation de ladite souche dans un bouillon nutritionnel afin d'obtenir le bouillon de culture;
b) optionnellement, séparation du bouillon de culture obtenu à l'étape a).

La fermentation aérobie dans un milieu liquide est préférée, comme dans le cas de la production d'autres antibiotiques, et la production des composés actifs à partir de la souche *Saccharothrix* SA 103 selon l'invention peut être effectuée à n'importe quelle température favorable à la croissance de cette souche, c'est-à-dire allant de la température ambiante jusqu'à 43 °C. De préference, on utilise une température allant de 25 °C à 32 °C. De manière encore préférée, la température est de 30 °C. Cette culture peut durer plusieurs jours, par exemple de 2 à 10 jours. Normalement, le pH est légèrement alcalin, mais le pH exact peut varier selon le milieu de culture utilisé. On prélève la souche après croissance sur gélose, notamment inclinée, et optionnellement stockée à faible température, et on l'inocule dans un milieu liquide conventionnel constitué de substances nutritives semblables à celles décrites plus haut pour la croissance de la souche *Saccharothrix* SA 103 selon l'invention, sous agitation de manière à obtenir un bouillon de culture.

La fermentation peut être réalisée dans des flacons Erlenmeyer et dans des fermentateurs industriels ou de laboratoire ayant diverses capacités. Lorsque l'on procède à la fermentation dans une cuve, il est souhaitable de produire un inoculum dans un bouillon nutritif en inoculant le bouillon nutritif avec un prélèvement d'une culture inclinée ou à plat, ou une culture lyophilisée de l'organisme. Après avoir obtenu de cette manière un inoculum, celui-ci est transféré de manière aseptisée dans le milieu de la cuve de fermentation pour une production à grande échelle des composés actifs. Le milieu dans lequel l'inoculum est produit peut être le même ou peut être différent de celui utilisé dans la cuve, pour autant qu'une croissance correcte du microorganisme soit obtenue.

Le bouillon nutritionnel selon l'invention contient de manière générale le même type de substances nutritives que celles du milieu de culture de la souche *Saccharothrix* SA 103 selon l'invention tel que décrit plus haut (source de carbone, source d'azote...). Il peut également contenir des agents anti-mousse tels que de la paraffine liquide, de l'huile de soja, des graisses ou du silicone.

L'étape b) optionnelle de séparation du bouillon de culture peut être effectuée par n'importe quel procédé bien connu de l'homme de l'art, seul ou associé à un autre procédé de séparation, tel que par exemple une centrifugation, une filtration ou une pasteurisation.

Ainsi, de référence, le procédé de production d'un bouillon de culture selon l'invention est **caractérisé en ce que** la sépararation réalisée à l'étape b) optionnelle est une centrifugation et/ou une filtration et/ou une pasteurisation.

L'invention a encore pour objet un bouillon de culture susceptible d'être obtenu selon le procédé selon l'invention.

A partir du bouillon de culture obtenu selon le procédé de production de l'invention; on procède à l'extraction des composés actifs. Pour cela, on utilise un solvant organique, tel que par exemple, mais sans s'y limiter, le n-butanol, l'acétate d'éthyle, le dichlorométhane, le n-propanol ou le 2-propanol.

Après l'étape d'extraction, la déshydratation de la phase organique peut être réalisée de manière facultative afin d'éliminer de nombreuses impuretés polaires dans le concentrat actif, ce qui facilitera ultérieurement la purification finale du concentrat actif par HPLC. Pour cela, on peut utiliser du sulfate de sodium anhydre ou du sulfate de magnésium, et/ou on procède au séchage *in vacuo* de la phase organique. L'utilisation d'une filtration sur gel (gel dextran réticulé), d'une chromatographie sur colonne cellulose, d'une résine échangeuse d'ions ou d'une chromatographie sur couche mince (gel de silice) peut également être envisagée.

De telles méthodes sont bien connues de l'homme de l'art, qui saura utiliser ces différentes techniques seules ou combinées, de la manière la plus appropriée en vue de récupérer un concentrat actif à partir du bouillon de culture de la souche *Saccharothrix* SA 103 selon l'invention.

Ainsi, encore un autre objet de la présente invention est un procédé de production d'un concentat actif à partir du bouillon de culture selon l'invention, **caractérisé en ce qu**'il comprend les étapes suivantes :
a) extraction organique du bouillon de culture avec un solvant organique ;
b) optionnellement, déshydratation de la phase organique obtenue et/ou séchage *in vacuo* ;
c) optionnellement, mise en suspension du concentrat actif, de préférence filtration de la suspension obtenue, et répétition des étapes a) et b) d'extraction organique et de déshydratation.

Le concentrat actif susceptible d'être obtenu selon le procédé de la présente invention constitue encore un objet de la présente invention.

Le concentrat actif ainsi obtenu est ensuite identifié au moyen d'analyses instrumentales telles que le spectre d'absorption des rayons visibles/ultraviolets, le spectre d'absorption des rayons infrarouges, le spectre RMN-¹H et le spectre RMN-¹³C, la spectrométrie de masse, et également l'analyse chromatographique (colonnes chromatographiques gel de silice ou gel dextran, résines échangeuses d'ions, chromatographie en phase liquide, chromatographie liquide haute performance en phase inverse ou HPLC, etc...). Ceci permet de caractériser et de produire les différents composés actifs, également dénommées « fractions actives », susceptibles d'être contenus dans le concentrat actif obtenu.

Ainsi, l'invention a encore pour objet un procédé de production d'un composé actif à partir du concentrat actif selon l'invention par chromatographie liquide haute performance en phase inverse (reverse phase HPLC), de préférence précédée d'une chromatographie sur couche mince et/ou d'une chromatographie liquide à basse pression.

Les composés actifs qui sont susceptibles d'être obtenus par le procédé de production selon l'invention correspondent donc à des éluats obtenus par chromatographie HPLC et sont quasiment purs.

La purification par chromatographie HPLC est indispensable pour séparer les composés actifs à partir du concentrat actif. La chromatographie sur couche mince et/ou la chromatographie liquide à basse pression telle que du type Sephadex LH 20 permet l'obtention d'un concentrat actif débarrassé de nombreuses impuretés et facilite ainsi la purification ultérieure par chromatographie HPLC.

De préférence, le procédé de production d'un composé actif selon l'invention est **caractérisé en ce que** le composé actif est une mutactimycine telle que la mutactimycine P11, la mutactimycine PR, la mutactimycine F ou la mutactimycine G, ou une aldgamycine telle que l'aldgamycine G, l'aldgamycine H on l'aldgamycine P10b, ou les sels d'addition pharmaceutiquement acceptables, isomères, énantiomères, diastéréoismères, ainsi que les mélanges de ces composés actifs.

La mutactimycine P11 possède la formule suivante :

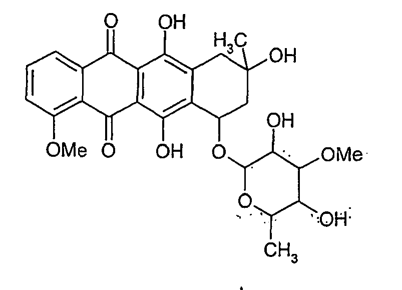

Elle a été identifiée comme correspondant à la mutactimycine C, déjà connue et enregistrée dans la base « Registry » sous le numéro RN 138689-81-3.

La mutactimycine PR possède la formule suivante :

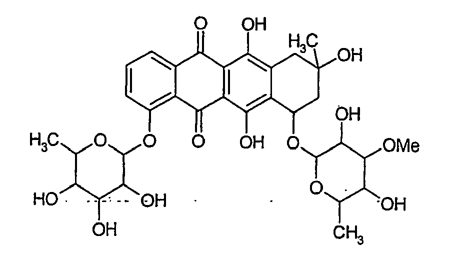

La mutactimycine F possède la formule suivante :

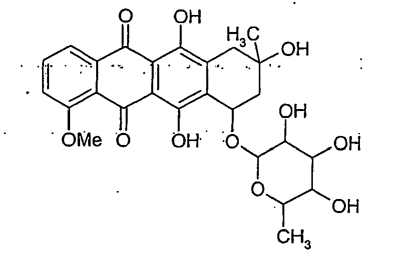

La mutactimycine G possède la formule suivante :

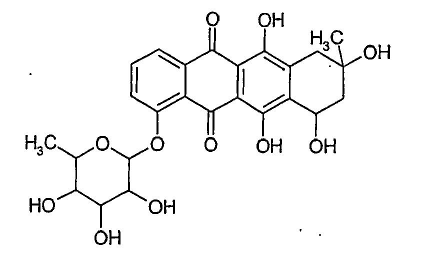

L'aldgamycine G (ou P10) possède la formule suivante :

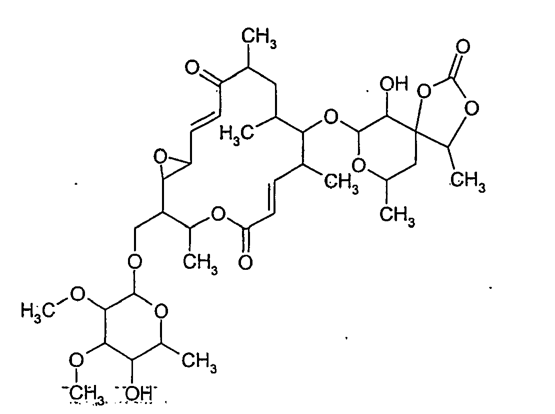

On connaît déjà l'aldgamycine de formule stéréochimique suivante, enregistrée dans la base « Registry » sous le numéro RN 107745-56-2.

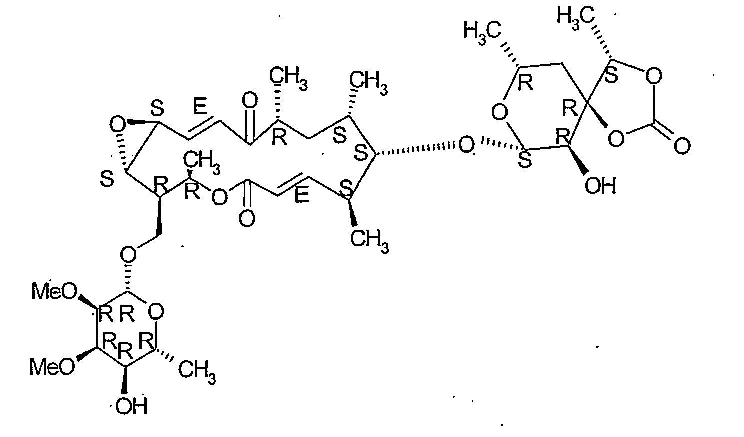

L'aldgamycine H (ou P8), possède la formule suivante :

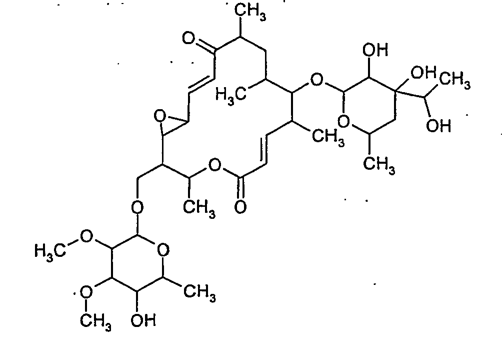

L'aldgamycine P10b (ou swalpamycine B) possède la formule suivante :

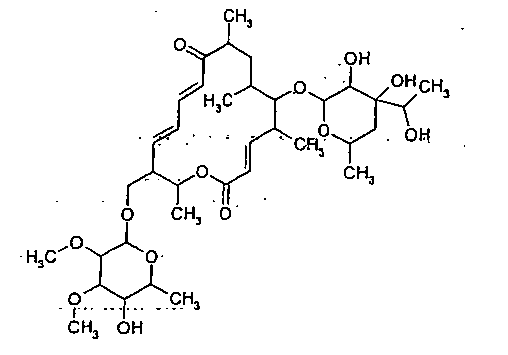

Les légendes des figures et exemples qui suivent sont destinées à illustrer l'invention sans aucunement en limiter la portée.

### LEGENDES DES FIGURES

Figure 1 : Profil de fermentation de la souche *Saccharothrix* sp. SA 103.
Figure 2 : Isolement et purification des produits actifs de la souche *Saccharothrix* sp. SA 103.

### EXEMPLES

### Exemple 1 : Matériels et méthodes

### 1.1 Micro-organisme

La souche productrice actinomycète *Saccharothrix* SA 103 selon l'invention a été isolée à partir d'un sol des régions arides de l'Algérie (Sahara) par mise en suspension d'un échantillon de sol dans de l'eau distillé stérile et dépôt sur une gélose humique-vitamines B contenant 50 µg/ml d'actidione. Une culture pure de la souche SA 103 a été préservée par lyophilisation. Elle a également été maintenue à 6°C à des fins d'utilisation au sein du laboratoire sur un milieu ISP N°2 incliné (Shirling et Gottlieb, Int. J. Syst. Bacteriol. 16 :313-340, 1996).

### 1.2 Taxonomie

Les caractéristiques taxonomiques de la souche SA 103 ont été déterminées par culture sur divers milieux décrits dans Shirling et Gottlieb (1996) et dans Waksman (« The actinomycetes », Vol. II, The Williams & Wilkins Co., Baltimore, 1961). Les caractéristiques morphologiques ont été déterminées après croissance à 30°C pendant 14 jours. Les noms de couleur et numéros de nuance ont été attribués en utilisant le système ISCC-NBS (« Inter Society Color Council » à l'intention du «National Bureau of Standards »). L'observation détaillée des morphologies du mycélium et des spores a été réalisée par microscopie électronique par balayage (Hitachi, modèle S-450). Les propriétés physiologiques ont été examinées à l'aide des méthodes Goodfellow (J. Gen. Microbiol. 69 :33-90, 1971) et Waksman (1961). Le type d'isomères de l'acide diaminopimélique dans la paroi cellulaire, et la composition cellulaire totale en sucres ont été déterminées par les méthodes de Becker et al. (Appl. Microbiol. 12 :421-423, 1964), et Lechevalier et Lechevalier. (« In The Actinomycetales », Ed. H. Prauser, pp. 311-316, Fisher Verlag, Jena, 1970). Les phospholipides et les acides mycoliques ont été analysés par la procédure de Minnikin et al. (Int. J. Syst. Bacteriol. 27 :104-107, 1977 ; J. Chromatography 188 :221-233, 1980).

### 1.3 Fermentation

Un prélèvement de la souche SA 103 à partir d'une culture inclinée mature a été inoculé dans un flacon Erlenmeyer contenant 50 ml de milieu de culture stérile contenant du glucose 0,4 %, un extrait de malt 1 % et un extrait de levure 0,4 % (ajusté au pH 7,2 avant stérilisation) et mis en culture sur un agitateur rotatif (250 rpm) à 30 °C pendant 2 jours. Pour la production d'antibiotiques, 3 ml du milieu de culture ont été transférés dans des flacons Erlenmeyer de 500 ml, chacun contenant 100 ml du milieu ci-dessus, et mis en culture pendant 10 jours en utilisant les mêmes conditions. La production de l'activité antibactérienne totale a été réalisée sur une gélose nutritive, par un test de diffusion sur gélose contre *Bacillus subtilis* ATCC 6633. L'inhibition de croissance a été examinée après 24 heures d'incubation à 30°C. L'activité antimicrobienne a été estimée en mesurant le diamètre de la zone d'inhibition. Le poids sec du mycélium a été déterminé dans des tubes Eppendorf remplis avec 1 ml de bouillon de culture homogénéisé et séché à 105°C pendant 24 heures (Pfefferle et al., J. Biotech. 80 :135-142, 2000).

### 1.4 Purification d'antibiotiques

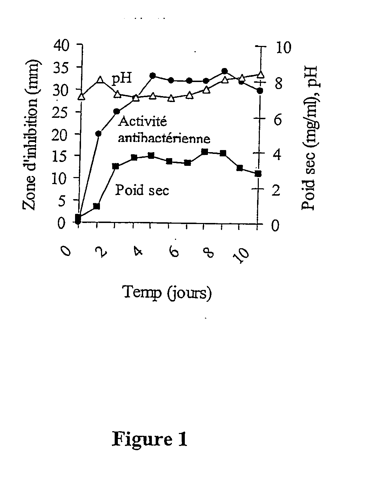

La production des antibiotiques antibactériens par la souche SA 103 est effectuée en milieu liquide MS + amidon (1 %) + extrait de levure (0,3 %) à pH 7,2 (100 ml de milieu par fiole Erlenmeyer de 500 ml, agitation à 250 rpm, incubation à 30 °C). Une cinétique a montré que la souche SA 103 commence à produire les antibiotiques dès le début de la phase exponentielle (1^{er} jour) et cette production se poursuit jusqu'au début de la phase stationnaire avant de se stabiliser pour décroître durant la phase de déclin (cf. Figure 1).
Au total, huit litres de culture ont été réalisées. Les filtrats son extraits au n-butanol. L'extrait butanolique contenant les antibiotiques antibactériens recherchés est retenu.
L'extrait butanolique, de couleur rouge, est concentré au Rotavapor. Dans un premier temps, une aliquote de cet extrait a été passé à travers une colonne de Séphadex LH 20 en utilisant comme phase mobile du méthanol dans de l'eau bidistillée (80 %). Cependant, la séparation des antibiotiques (visualisation à l'oeil nu et par antibiographie) n'a pas été concluante. Cette étape a donc été supprimée et l'extrait butanolique a été purifié directement par HPLC en phase inverse en utilisant une colonne C18 et des conditions isocratiques (63 % de méthanol dans de l'eau), un débit de 1,5 ml/min et une détection à 220 nm. Les fractions correspondant à tous les pics obtenus sur le profil ont été recueillies séparément, concentrées et testées contre *Bacillus subtilis.* Sept fractions (7 pics) se sont révélées actives: quatre de couleur rouge vif (P11, PR, F et G) et trois non colorées (P8, P10a et P10b). Ces antibiotiques sont purifiés par HPLC après 3 à 4 réinjections dans les mêmes conditions que précédemment.

### Exemple 2 : Résultats et discussion - Caractérisation taxonomique de la souche productrice

### 2.1 Morphologie de la souche

La souche SA 103 a formé un mycélium aérien rose bien développé qui se fragmentait en chaînes de spores droites ou flexibles. Les spores étaient en forme de bâtonnets et avaient une taille de 1,9-2,9x0,6-0,7 µm avec une surface lisse (Figure 1). Les endospores, les granules sclérales, les synnemata et les spores à flagelles n'ont pas été observés. Le mycélium substrat était rouge brûnatre à rouge foncé et ne montrait pas ou peu de fragments. La souche a produit un pigment rouge foncé abondant caractéristique qui a été révélé comme correspondant à des antibiotiques antibactériens.

### 2.2 Caractéristiques structurales

Le tableau 1 montre les caractéristiques de culture de la souche SA 103 sur différents supports de culture. La croissance de la souche était abondante sur gélose d'extrait de levure et d'extrait de malt, gélose Bennett et gélose nutritive mais était modérée sur gélose flocons d'avoine et gélose sels inorganiques - amidon. La couleur du mycélium rentrait dans la gamme entre rose jaunâtre à marron rougeâtre clair pour les hyphes aériens et orange brûnatre à rouge très foncé pour le mycélium substrat. La souche produisait un pigment soluble rouge foncé ou orange brûnatre sur tous les milieux utilisés, mais aucun pigment mélanoïdé n'a été observé.
L'espèce la moins différente est *Saccharothrix syringae* qui possède un mycélium aérien blanchâtre-rosâtre, un mycélium du substrat et des pigments solubles violet-rouge-brun.

### 2.3 Chimiotaxonomie

L'étude chimiotaxonomique a montré la présence d'acide *meso*-diaminopimélique et l'absence de glycine (paroi cellulaire de type III). Le motif en sucres de l'ensemble de la cellule a consisté en du rhamnose et du galactose (sucre cellulaire de type E) (Kroppenstedt, « The genus Nocardiopsis. In the Procaryotes » Ed., A. Balows et al., pp. 1139-1156, Springer Verlag, Berlin, 1192) et le phospholipide caractéristique était le phosphatidyl-éthanolamine (phospholipides de type PII). Aucun acide mycolique n'a été détecté.

### 2.4 Physiologie de la souche

Les résultats des tests physiologiques sont rapportés dans le tableau 2.
La souche est capable d'utiliser plusieurs composés organiques (caséine, gélatine, tween 80, amidon, tyrosine, etc.) dont la majorité des sucres. Par contre, à l'exception du mannitol, les dérivés alcooliques des oses (inositol, adonitol, dulcitol, érythritol et sorbitol) ne sont pas dégradés. La souche est capable de croître à 20 °C et 48 °C (avec un optimum à 30°C) et à pH 5 et 9 (avec un optimum entre 7 et 8). Elle est résistante au lysozyme et au violet cristal mais est sensible à tous les antibiotiques testés (11) à l'exception de la pénicilline et de la rifampicine.
La souche SA 103 se distingue de *Sa. syringae* par sa capacité à dégrader le laotose mais pas l'hypoxanthine et le butyrate de sodium, par sa sensibilité à lérythromycine, la gentamicine, l'oxytétracycline et la vancomycine, par sa résistance à la pénicilline, la rifampicine et le violet cristal et par sa croissance à pH 5.
De ce fait, la souche SA 103 pourrait être une nouvelle espèce de *Saccharothrix*
ou éventuellement une nouvelle sous-espèce de *Sa. syringae*.

### 2.5 Classification

En se basant sur les propriétés morphologiques et chimiques décrites plus haut, il a été considéré que la souche SA 103 appartenait au genre *Saccharothrix* (Labeda, et al, Int. J. Syst. Bacteriol. 34 :426-431, 1984). Comparée à l'espèce la plus proche *Saccharothrix syringae* NRRL B-16 468^{T}, la souche SA 103 différait par la capacité à dégrader le lactose mais pas l'hypoxanthine et le butyrate de sodium, par une susceptibilité à l'érythromycine, la gentamycine, l'oxytétracycline et la vancomycine, par une résistance à la pénicilline, la rifampicine, le cristal violet et l'azoture de sodium, et par la croissance à pH 5,0. Ainsi, la souche a été désignée *Saccharothrix* sp. SA 103.

### 2.6 Fermentation

L'évolution au cours du temps de la production de l'activité antibactérienne par *Saccharothrix* sp. SA 103 est montrée dans la figure 1. La production de l'activité biologique contre *B. subtilis* a commencé lors du premier jour et était à son maximum au jour 4 pour devenir stable. La biomasse a augmenté lors des trois premiers jours, est restée stable, puis a diminué après le jour 8. La cinétique du pH a montré une augmentation remarquable lors du premier jour, puis est redevenue neutre, et a augmenté à la fin de la fermentation. De manière générale, la production des métabolites secondaires par les micro-organismes intervient pendant la phase stationnaire mais dans le présent cas la production de l'activité biologique était fortement corrélée à la croissance et était observée pendant toute l'évolution au cours du temps. La même cinétique de production au cours du temps a été observée pour la production d'antibiotiques dithiolopyrrolone par *Saccharothrix* sp. SA 233 (Lamari et al., J. Antibiotics 55 :696-701, 2002) et la production d'acide clavulanique par *Streptomyces clavuligerus* (Lebrihi et al., Appl. Microbiol. Biotechnol. 26 :130-135, 1987).

### 2.7 Détermination de la structure des antibiotiques

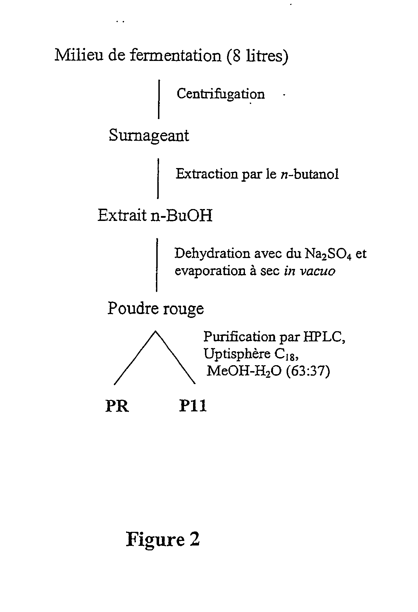

Les différentes étapes d'isolement et de purification d'antibiotiques sont résumés dans la figure 2.

### 2.7.1 Antibiotiques de la famille des anthracyclines

Les antibiotiques P11, PR, F et G sont de couleur rouge vif à pH neutre, jaune à pH acide et violet-bleu à pH basique. P11 est l'antibiotique majoritaire et PR, l'antibiotique minoritaire. Ces antibiotiques ont fait l'objet d'études spectroscopiques suivantes: UV visible, infra-rouge, spectrométrie de masse et RMN du proton et du carbone 13 (avec études des corrélations). Des tests complémentaires, tels que la solubilité, la détermination des Rf par CCM et l'analyse élémentaire (cette dernière uniquement pour P11) ont été également effectués.

### Identification de la molécule P11 à la mutactimycine C

La molécule P11 possède un poids moléculaire de 530 et la formule chimique C₂₇H₃₀O₁₁. Les maxima obtenus dans l'UV-visible (MeOH) sont: 219, 234, 250, 287, 478, 496 et 531 nm. Le spectre est similaire à celui des antibiotiques de la famille des anthracyclines, de même que les changements de couleur à différents pH. Le spectre infra-rouge suggère la présence d'aromatiques, de groupements hydroxyles, méthyles et méthoxyles. Cependant, la structure finale a été élucidée après une étude détaillée par RMN du proton et du carbone 13 (déplacements chimiques, constantes de couplage spin-spin, intensités des signaux et corrélations ¹H-¹H cosy 45, ¹H-¹³C HMCQ et ¹H-¹³C HMBC). La molécule P11 a pu ainsi être identifiée à la mutactimycine C, connue pour être sécrétée par un mutant de *Streptomyces* sp. La mutactimycine C est constituée par le noyau anthracycline (4 cycles accolés dont un quinonique compris entre deux benzéniques), relié (par le biais du carbone C7) à un ose (le 6-deoxy-3-O-methyl-α-mannopyranoside). P11 est très soluble dans l'eau, le méthanol, le n-butanol, l'éthanol, le 1- propanol et l'acétone et insoluble dans le n-hexane et le toluène.

### Détermination de la structure chimique de nouvelles mutactimycines

### Mutactimycine PR

La molécule mineure PR possède un poids moléculaire de 662 qui correspond à la formule C₃₂H₃₈O₁₅. Les maxima obtenus dans l'UV-visible et les bandes dans l'infra-rouge sont identiques à ceux dé P11 et les fragments de masse assez proches entre les deux antibiotiques, ce qui suggère une forte ressemblance dans la structure chimique. PR est très soluble dans l'eau, le méthanol et l'éthanol et insoluble dans le chloroforme, le dichlorométhane, l'acétate d'éthyle, le n-hexane et le toluène. La RMN du proton et celle du carbone 13 ont permis d'élucider la structure de PR qui s'est révélée être proche de P11 (mutactimycine C) avec comme seule différence la présence d'un second ose lié au carbone 4 du 1^{er} cycle benzénique (à la place de -OCH₃). Le second ose est le 6-deoxy-mannopyranoside (différence avec le premier par l'absence d'un méthyle). De par cette structure, la molécule PR appartient à la famille des anthracyclines et au groupe des mutactimycines. Cependant, elle diffère de toutes les mutactimycines connues et représente donc un nouvel antibiotique de ce groupe, nommé mutactimycine PR.

### Mutacthmycine F

L'antibiotique F a un poids moléculaire de 516 (soit 14 de moins que P11) correspondant à une formule moléculaire C₂₆H₂₈O₁₁. Ses spectres UV-visible et infra-rouge et ses fragments de masse sont très proches de ceux de P11 et PR. Ceci suggère donc une forte ressemblance entre les trois molécules. Le composé F est soluble dans l'eau, le n-butanol, le 1-propanol et l'éthanol et insoluble dans le n-hexane et le toluène. Les RMN du proton et du carbone 13 ont permis d'établir la structure de la molécule qui s'est révélée être proche de P11 (mutactimycine C), mais avec comme seule différence la présence d'un OH sur le carbone C3 du résidu mannopyranosyl, au lieu d'un groupement -OCH3. L'antibiotique F est différent de toutes les mutactimycines connues et représente une nouvelle molécule dénommée mutactimycine F.

### Mutactimycine G

La molécule possède un poids moléculaire de 502 correspondant à une formule C₂₅H₂₆O₁₁. De fortes similitudes entre G, F, PR et P11 sont observées dans les spectres UV-visible et infra-rouge, ainsi que les fragments de masse. Le produit G est soluble dans l'eau, le n-butanol, le 1-propanol, le méthanol et l'éthanol et insoluble dans le chloroforme, le dichlorométhane, l'acétate d'éthyle, le n-hexane et le toluène. Les RMN du proton et du carbone 13 ont permis d'élucider la structure de cette molécule qui s'est révélée être proche de PR, avec comme seule différence l'absence du second ose lié au carbone C7 du 4^{éme} cycle du noyau anthracycline. L'antibiotique G représente donc une nouvelle molécule du groupe des mutactimycines et a ainsi été appelé mutactimycine G.

### 2.7.2 Antibiotiques de la famille des macrolides

Les antibiotiques P8, P10a et P10b ne sont pas colorés. P8 est la molécule majoritairement produite par *Saccharothrix* sp. SA 103 après P11 (mutactimycine C). Les trois antibiotiques sont très solubles dans le méthanol, le n-butanol, l'éthanol, le 1 et 2-propnol, l'acétate d'éthyle, l'acétone, le chloroforme et le dichlorométhane, mais insolubles dans l'eau et le n-hexane. Ces antibiotiques ont fait l'objet des mêmes études spectroscopiques que les mutactimycines décrites précédemment, ce qui a permis d'aboutir à la détermination de leurs structures chimiques.

### Identification de P10a à l'aldgamycine G

L'antibiotique P10a possède un poids moléculaire de 740 correspondant à la formule moléculaire C₃₇H₅₆O₁₅. Le spectre UV-visible et très semblable à celui des aldgamycines et a montré un maximum à 216 et deux épaulements à 245 et 278 nm. Le spectre infra-rouge présente plusieurs bandes d'absorptions qui indiquent la présence de groupements méthyle, méthoxyles, hydroxyles et une fonction carbonate. P10a est soluble dans le n-butanol, le 1 et 2-propanol, le méthanol, le dichlorométhane, l'acétate d'éthyle, le chloroforme, l'acétone et l'éthanol et insoluble dans le n-hexane, le toluène et l'eau. La molécule P10a a pu ainsi être identifiée à l'aldgamycine G, connue pour être sécrétée par *Streptomyces avidinii*. L'aldgamycine G est un macrolide neutre dont le cycle lactonique comporte 16 atomes sur lequel son reliés deux sucres méthylés, le mycinoe en position C14 et l'aldgarose en position C5.

### Détermination des structures chimiques des nouveaux macrolides

### Aldgamycine H

L'antibiotique P8 possède un poids moléculaire de 714, soit une formule moléculaire C₃₆H₅₈O₁₄. Ses propriétés physico-chimiques sont très semblable à celles de P10a (aldgamycine G). Cependant, le spectre UV-visible de P10b présente une intensité d'absorbance moindre à 245 et 278 nm et son spectre infra-rouge ne contient plus la bande d'absorption à 1800 cm⁻¹ caractéristique du groupement carbonate comme c'est le cas de l'aldgamycine G. Le produit P8, après analyse de toutes ses données spectroscopiques a été identifié à une nouvelle aldgamycine nommée H qui se rapproche de l'aldgamycine E, mais elle diffère par l'absence de la fonction carbonate sur le sucre aldgarose qui est hydrolysée dans ce cas.

### Swalpamycine B

La molécule P10b n'a pu être séparée du produit P10a par HPLC et de ce fait, elle a été analysé en même temps que le produit P10a sous forme de complexe. Elle possède un poids moléculaire de 698 et la formule chimique C₃₆H₅₈O₁₃. Les analyses RMN du proton et du carbone 13 ont permis d'élucider la structure finale. Ainsi P10a possède une structure chimique très semblable à celle de P8 et de la swalpamycine.

### 2.8 Activités microbiologiques des antibiotiques

L'activité microbiologique des mutactimycines P11, PR, F et G et des macrolides P8, P10(a et b) est dirigée surtout contre les bactéries à Gram positif. Les bactéries les plus sensibles sont *Micrococcus luteus* et *Klebsiella pneumoniae* qui est la seule bactérie à Gram négatif à être sensible.
Les concentrations minimales inhibitrices (CMI) sont de 5 µg/ml pour *M. luteus* et *K. pneumoniae*, 10 µg/ml pour *Staphylococcus aureus* CIP 53156, 40 µg/ml pour *Bacillus subtilis*, 50 µg/ml pour *Listeria monocytogenes* et 75 µl/ml pour *Mycobacterium smegmatis*. Les nouvelles molécules PR, F et G sont actives contre les mêmes microorganismes (à l'exception de *L. monocytogenes*).
L'ensemble des molécules n'a aucune action contre *S. aureus* CIP 7625, les bactéries à Gram négatif *Escherichia coli, Pseudomonas syringae* pathovar *syringae* et *Agrobacterium tumefasciens* et contre la levure *Saccharomyces cerevisiae* et le champignon filamenteux *Mucor romannianus*.

L'activité des macrolides (aldgamycines G et H) et swalpamycine B est plus importante que celles des mutactimycines. En effet, les CMI ne sont que de 0,1 µg/ml pour *K. pneumonie*, 1 µg/ml pour *Bacillus subtilis*, *Micrococcus luteus* et *Staphylococcus aureus* CIP 53156 qui sont les plus sensibles et respectivement 30 et 50 µg/ml pour *Listeria monocytogenes* et *Mycobacterium smegmatis*. Les bactéries à Gram négatif (sauf *K. pneumoniae)*. et les champignons sont résistants.

| **Test organisms** | **MIC (ug/ml) (swalpamycine)** |
|---|---|
| *Bacillus subtilis* ATCC 6633 | 1 |
| *Micrococcus luteus* ATCC 9314 | 1 |
| *Staphylococcus aureus* CIP 7625 | > 100 |
| *Staphylococcus aureus* CIP 53156 | 1 |
| *Listeria monocytogenes* CIP 82110 | 30 |
| *Mycobacterium smegmatis* ATCC 607 | 50 |
| *Klebsiellapneumoniae* CIP 82.91 | 0.1 |
| *Escherichia coli* ATCC 10536 | > 100 |
| *Pseudomonas syringae* No 1882 | > 100 |
| *Agrobacterium tumefasciens* No 2410 | > 100 |
| *Mucor ramannianus* NRRL 1829 | > 100 |
| *Saccharomyces cerevisiae* ATCC 4226 | > 100 |

| **Test organisms** | **MIC (µg/ml) of (aldgamycine H)** |
|---|---|
| *Bacillus subtilis* ATCC 6633 | 10 |
| *Micrococcus luteus* ATCC 9314 | 1 |
| *Staphylococcus aureus* CIP 7625 | > 100 |
| *Staphylococcus aureus* CIP 53156 | 5 |
| *Listeria monocytogenes* CIP 82110 | > 100 |
| *Mycobacterium smegmatis* ATCC 607 | 20 |
| *Klebsiella pneumoniae* CIP 82.91 | 0.5 |
| *Escherichia coli* ATCC 10536 | >100 |
| *Pseudomonas syringae* No 1882 | > 100 |
| *Agrobacterium tumefasciens* No 2410 | >100 |
| *Mucor ramannianus NRRL* 1829 | >100 |
| *Saccharomyces cerevisiae* ATCC 4226 | >100 |

**Tableau 1 : Caractéristiques culturales de Saccharothrix sp. SA 103 .**

| Milieu | Croissance | Mycélium aérien | Mycélium du substrat | Pigment diffusible |
|---|---|---|---|---|
| Extrait de levure-extrait de malt agar (ISP No 2) | Bonne | Abondant Modéré Rose jaunâtre (29) | Rouge très sombre (14) | Rouge noirâtre (16) |
| | | | | |
| Farine d'avoine agar (ISP No 3) | Modérée | Modéré Rose jaunâtre pale (31) | Orange brunâtre (54) | Orange brunâtre (54) |
| | | | | |
| Sels inorganiqu e-amidon agar (ISP No 4) | Modérée | Modéré Rose brunâtre (33) | Orange brunâtre (54) | Orange brunâtre (54) |
| | | | | |
| Bennett agar | Bonne | Abondant Brun rougeâtre clair (42) | Brun rougeâtre sombre (44) | Brun rougeâtre sombre (44) |
| | | | | |
| Gélose nutritive | Bonne | Modéré à abondant Rose jaunâtre (29) | Rouge foncé (13) | Rouge foncé (13) |
| | | | | |

**Tableau 2 : Caractéristiques physiologiques de la souche Saccharothrix sp. SA 103.**

| | | | |
|---|---|---|---|
| Dégradation de | | Réduction de nitrate | + |
| Adénine | - | Production de pigments | |
| Arbutine | + | mélanoides | - |
| Caséine | + | | |
| Gélatine | + | Décarboxylation du sodium : | + |
| Esculine | + | Acétate | - |
| Guanine | - | Benzoate | - |
| Hypoxanthine | - | Butyrate | + |
| Amidon | + | Citrate | - |
| Testostérone | + | Oxalate | + |
| Tween 80 | + | Propionate | + |
| Tyrosine | + | Pyruvate | + |
| Xanthine | - | Succinate | - |
| Adonitol | - | Tartrate | |
| L-Arabinose | + | Croissance à : | + |
| Cellobiose | + | 48°C | + |
| Dextrine | + | pH 5 | + |
| Dulcitol | - | pH 9 | |
| Erythritol | - | Tolérance au : | + |
| D-Fructose | + | Cristal violet (0.001%) | + |
| Galactose | + | Lysozyme (0.005%) | + |
| D-Glucose | + | Phénol (0.05%) | - |
| Glycérol | + | Phénol (0.1%) | + |
| Inositol | - | Tellurite de potassium (0.01 %) | + |
| Lactose | - | Azide de sodium (0.001%) | - |
| Maltose | + | Azide de sodium (0.01%) | - |
| D-Mannitol . | + | Chlorure sodium (5%) | |
| D-Mannose | + | Résistance au : | - |
| Mélézitose | - | Chloramphénicol (25 µg/ml) | - |
| Mélibiose | - | Cyclosérine (10 µg/ml) | - |
| α-Méthyle-D-glucoside | - | Erythromycine (10 µg/ml) | - |
| D-Raffinose | - | Gentamicine (10 µg/ml) | - |
| L-Rhamnose | + | Kanamycine (25 µg/ml) | - |
| Ribose | + | Novobiocine (10 µg/ml) | + |
| Sorbitol | - | Oxytétracycline (25 µg/ml) | + |
| Saccharose | + | Pénicilline (25 µg/ml) | - |
| Tréhalose | + | Rifampicine (5 µg/ml) | - |
| D-Xylose | + | Streptomycine (10 µg/ml) | - |
| | | Vancomycine (5 µg/ml) | |

**Tableau 3 : Spectre antimicrobien des antibiotiques PR et P11 :**

| Souches test | CMI (µg/ml) | |
|---|---|---|
| | PR (1) | P11 (2) |
| *Bacillus subtilis* ATCC 6633 | 75 | 40 |
| *Micrococcus luteus* ATCC 9314 | 50 | 5 |
| *Staphylococcus aureus* CIP 7625 | >100 | >100 |
| *Staphylococcus aureus* CIP 53156 | 50 | 10 |
| *Listeria monocytogenes* CIP 82110 | >100 | 50 |
| *Mycobacterium smegmatis* ATCC 607 | >100 | 75 |
| *Klebsiella pneumoniae* CIP 82.91 | 40 | 5 |
| *Escherichia coli* ATCC 10536 | >100 | >100 |
| *Pseudomonas syringae* No 1882 | >100 | >100 |
| *Agrobacterium tumefaciens* No 2410 | >100 | >100 |
| *Mucor ramannianus* NRRL 1829 | >100 | >100 |
| *Saccharomyces cerevisiae* ATCC 4226 | >100 | >100 |

**Tableau 4. Propriétés physicochimiques des produits PR et P11 (mutactimycine).**

| | | **mutactimycine PR** | **P11 (mutactimycine C)** |
|---|---|---|---|
| Apparence | | Poudre rouge | Poudre rouge |
| Couleur dans H₂O | | | |
| | Acide | Jaune | Jaune |
| | Neutre | Rouge | Rouge |
| | Basique | Violet-bleu | Violet-bleu |
| | | | |
| Formule chimique | | C₃₂H₃₈O₁₅ | C₂₇H₃₀O₁₁ |
| | | | |
| Poids moléculaire | | 662 | 530 |
| | | | |
| Nano-ESI-MS (*m*/*z*) | | | |
| | Mode négatif | 660.8 [M-H]⁻, 514.9, 354, 337, 319.1, 291.1 | 528.8 [M-H]⁻, 351, 333, 315, 294.2, 293.1 |
| | Mode positif | 685.2 [M+Na]⁺ 539, 507, 360.9, 342.9 | 553.1 [M+Na]⁺, 375, 356.9, 198.3 |
| | | | |
| UV λₘₐₓ nm dans MeOH | | 219, 234, 250, 287, 478, 496, 531 | 219, 234, 250, 287, 478, 496, 531 |
| | | | |
| IR vₘₐₓ Cellule de diamant (cm⁻¹) | | 3393, 2969, 2932,2878 2841,2709,2360,2113, 1611, 1583, 1444, 1408, 1379, 1352, 1285, 1238, 1213, 1133, 1110, 1070, 1048 | 3393, 2969, 2932,2878 2841,2709,2360,2113, 1611, 1583, 1444, 1408, 1379, 1352, 1285, 1238, 1213, 1133, 1110, 1070, 1048 |
| | | | |
| Solubilité relative | | | |
| | Très soluble | MeOH, EtOH, H₂O | MeOH, Me₂CO, H₂O, |
| | | | *n*-BuOH, EtOH, |
| | | | *1*-PrOH |
| | Moyennement soluble | Me₂CO, *n*-BuOH, | CH₂Cl₂, CHCl₃, EtOAc, |
| | | *1*-PrOH, *2*-PrOH | 2-PrOH |
| | | | |
| | Insoluble | CH₂Cl₂, CHCl₃ EtOAc, *n*-hexane, toluène | *n*-hexane, toluène |
| | | | |
| CCM (Valeur des Rf)^{a} | | | |
| | (I) | 0.16 | 0.44 |
| | (II) | 0.58 | 0.64 |
| | (III) | 0.82 | 0.80 |
| | | | |
| HPLC (Rt)^{b} | | 31.74 min | 35.65 min |

| | | | |
|---|---|---|---|
| ^{a}CCM sur gel de silice (MerckNo 5715). (I): EtOAc-MeOH (100:15). (II): *n*-BuOH-CH₃COOH-H₂O (3:1:1). (III):MeOH-CH₂Cl₂ (4:1). ^{b}Conditions d'HPLC: Uptisphere C₁₈ UP5ODB (250x7.8 mm, d.i.), Phase mobile: isocratique à 63% MeOH in H₂O, Débit: 1.5 ml/min, Détection: UV (220 nm). | | | |

**Tableau 5. Assignements des données RMN du ¹H et du ¹³C du produit PR dans du DMSO-d6 à 298 K et dans du DMF-d₇ à 278 K.**

| Position | DMSO | | DMF | |
|---|---|---|---|---|
| | δ_{H} | δ_{C} | δ_{H} | δ_{C} |
| 1 | 8.00 (d) | 121.0 | 7,96(d) | 120.6 |
| 2 | 7.84 (br dd) | nd | 7.81 (dd) | 135.3 |
| 3 | 7.66 (br) | nd | 7.68 (d) | 124.1 |
| 4 | | nd | | 157.9 |
| 4a | | nd | | 122.6 |
| 7 | 4.89 (m) | 73.2 | 4.92 (m) | 73.7 |
| 8 | 2.10 (dd) / 1.92 (dd) | 43.3 | 2.16 (dd) / 1.98 (dd) | 43.6 |
| 9 | | 67.8 | | 67.7 |
| 10 | 2.83 (d) / 2.61 (d) | 38.3 | 2.84 (d) / 2.65 (d) | 38.2 |
| 12a | | nd | | 136.0 |
| 6-OH | nd | | nd | |
| 9-OH | 4.70 (br) | | nd | |
| 9-Me | 1.30 (s) | 29.7 | 1.32 (s) | 29.2 |
| 11-OH | nd | | nd | |
| 1' | 5.16 (br s) | 104.4 | 5.22 (br s) | 104.7 |
| 2' | 3.83 (m) | 67.0 | 3.92 (m) | 67.1 |
| 3' | 3.00 (dd) | 81.5 | 3.04 (dd) | 81.7 |
| 4' | 3.33 ^{a} | 71.0 | 3.44 (dd) | 72.0 |
| 5' | 3.64 (dq) | 70.2 | 3.70 ^{a} | 69.8 |
| 2'-OH | 4.88 | | nd | |
| 3'-OMe | 3.22 (s) | 56.9 | 3.17 (s) | 56.6 |
| 4'-OH | 4.90 | | nd | |
| 5'-Me | 1.21 (d) | 18.7 | 1.17 (d) | 18.0 |
| 1" | 5.69 (br s) | 99.1 | 5.73 (br s) | 99.4 |
| 2" | 4.00 (m) | 71.0 | 4.09 (m) | 71.3 |
| 3" | 4.04 (dd) | 70.9 | 4.10 (dd) | 71.3 |
| 4" | 3.35 ^{a} | 72.4 | 3.42 (dd) | 72.2 |
| 5" | 3.52 (dq) | 70.9 | 3.54 ^{a} | 70.8 |
| 2"-OH | 5.15 (br) | | nd | |
| 3"-OH | 4.89 (br) | | nd | |
| 4"-OH | 4.97 | | nd | |
| 5"-Me | 1.09 (d) | 18.7 | 1.05 (d) | 17.9 |

| | | | | |
|---|---|---|---|---|
| Seul les signaux détectés sont représentés. ^{a}Signal sous le HOD résiduel. nd: non détecté. | | | | |

**Tableau.6. Assignements des données RMN du ¹H et du ¹³C du produit P11 dans du DMSO-d6 à 298 K.**

| **Position** | δ_{H} | δ_{C} |
|---|---|---|
| 1 | 7.81 (m) | 119.4 |
| 2 | 7.83 (m) | 136.0 |
| 3 | 7.56 (m) | 119.1 |
| 4 | | 161.5 |
| 4a | | 120.8 |
| 5 | | 186.9 |
| 5a | | 111.1 |
| 6 | | 157.4 |
| 6a | | 136.0 |
| 7 | 4,86 (dd) | 73.5 |
| | 2.13 (dd) / 1.93 | |
| 8 | (dd) | 42.8 |
| 9 | | 68.2 |
| 10 | 2.76 (d) / 2.62 (d) | 37.4 |
| 10a | | 136.8 |
| 11 | | 155,4 |
| 11a | | 111.4 |
| 12 | | 187.4 |
| 12a | | 135.7 |
| 4-OMe | 3.95 (s) | 56.2 |
| 6-OH | 14.13 (s)^{a} | |
| 9-OH | 4.78 (s) | |
| 9-Me | 1.31 (s) | 28.1 |
| 11-OH | 13.19 (s)^{a} | |
| 1' | 5.12 (d) | 104.2 |
| 2' | 3.87 (m) | 67.1 |
| 3' | 3.02 (dd) | 81.2 |
| 4' | 3.32 (ddd) | 71.7 |
| 5' | 3.63 (dq) | 69.8 |
| 2'-OH | 4.82 (d) | |
| 3'-OMe | 3.23 (s) | 56.1 |
| 4'-OH | 4.91 (d) | |
| 5'-Me | 1.21 (d) | 17.0 |

| | | |
|---|---|---|
| ^{a}Signaux pouvant être interchangeables. | | |

**Tableau 7. Propriétés physico-chimiques du composé F.**

| | | **Mutactimycine F** |
|---|---|---|
| apparence | | Poudre rouge vif |
| Formule chimique | | C₂₆H₂₈O₁₁ |
| Poids moléculaire | | 516 |
| Nano-ESI-MS (*m*/*z*) | | |
| | Mode négatif | 514.9 [M-H]⁻ |
| | | |
| | Mode positif | 539.11 [M+Na]⁺ |
| | | |
| UV λₘₐₓ nm ans MeOH | | 219, 234, 252, 286, 473, 494, 531 |
| | | |
| IR νₘₐₓ Cellule de diamant (cm⁻¹) | | 3351, 2970, 2927,2854, 2360, 2336, 2114, 1981, 1798, 1610, 1582, 1444, 1406, 1381, 1356, 1271, 1240, 1215, 1137, 1091, 1069, 1048 |
| | | |
| Solubilité relative | | |
| | Très soluble | *n*-BuOH, *1*-PrOH |
| | Moyennement soluble | MeOH, EtOH |
| | Insoluble | H₂O, CH₂Cl₂, CHCl₃ EtOAc, Me₂CO, *n*-hexane, toluene |
| | | |
| CCM (valeur des Rf)^{a} | | |
| | (I) | 0.30 |
| | (II) | 0.62 |
| | (III) | 0.78 |
| | | |
| HPLC (Rt)^{b} | | 22.25 min |

| | | |
|---|---|---|
| ^{a}CCM sur gel de silice (Merck No 5715). (I): EtOAc-MeOH (100:15). (II): *n-*BuOH-CH₃COOH-H₂O (3:1:1). (III): MeOH-CH₂Cl₂ (4:1). ^{b}Conditions d'HPLC: Uptisphere C₁₈ UP5ODB (250x7.8 mm, d.i.), Phase mobile: isocratique à 63% MeOH in H₂O, Débit: 1.5 ml/min, Détection: UV (220 nm). | | |

**Tableau 8. Assignements des données RMN du ¹H et du ¹³C du produit F dans du DMSO-d6 à 298 K.**

| **Position** | δ_{H} | δ_{C} |
|---|---|---|
| 1 | 7.91 (m) | 120.5 |
| 2 | 7.90 (m) | 137.0 |
| 3 | 7.64 (m) | 119.8 |
| 4 | | 161.6 |
| 4a | | 119.8 |
| 5 | | 187.4 |
| 5a | | 111.4 |
| 6 | | 157.5 |
| 6a | | 137.3 |
| 7 | 4.90 (dd) | 73.2 |
| 8 | 2.13 (dd) / 1.94 (dd) | 43.3 |
| 9 | | 67.8 |
| 10 | 2.83 (d) / 2.64 (d) | 38.3 |
| 10a | | 137.0 |
| 11 | | 155.5 |
| 11a | | 111.5 |
| 12 | | 187.3 |
| 12a | | 135.7 |
| OMe-4 | 3.99 (s) | 57.5 |
| OH-6 | 14.16 (br s)^{a} | |
| OH-9 | 4.78 (s) | |
| Me-9 | 1.30 (s) | 29.8 |
| OH-11 | 13.26 (br s)^{a} | |
| 1' | 5.07 (d) | 104.6 |
| 2' | 3.61 (m) | 71.4 |
| 3' | 3.30 (ddd) | 71.5 |
| 4' | 3.24 (ddd) | 72.7 |
| 5' | 3.60 (dq) | 70.2 |
| OH-2' | 4.78 (d) | |
| OH-3' | 4.47 (d) | |
| OH-4' | 4.77 (d) | |
| Me-5' | 1.20 (d) | 19.8 |

| | | |
|---|---|---|
| ^{a}Signal sous le HOD résiduel. nd: non détecté. | | |

**Tableau 9. Propriétés physico-chimiques du composé G.**

| | | **G** |
|---|---|---|
| Apparence | | Poudre rouge vif |
| Formule chimique | | C₂₅H₂₆O₁₁ |
| Poids moléculaire | | 502 |
| Nano-ESI-MS (*m*/*z*) | | |
| | Mode négatif | 500.9 [M-H]⁻, 355, 337, 319.2, 291.2 |
| | | |
| UV λₘₐₓ nm dans MeOH | | 219,234,252,286,473,494,531 |
| | | |
| IR νₘₐₓ Cellule de diamant | | 3351, 2970, 2927,2854, 2360, 2336, 2114, 1981, 1798, 1.610, 1582, 1444, 1406, 1381, 1356, 1271, 1240, 1215, 1137, 1091, 1069, 1048 |
| | (cm⁻¹) | |
| | | |
| Solubilité relative | | |
| | Très soluble | *n*-BuOH, *1*-PrOH, |
| | | |
| | Moyennement soluble | MeOH, EtOH, |
| | | |
| | Insoluble | H₂O, CH₂Cl₂, CHCl₃ EtOAc, Me₂CO, *n*-hexane, toluène |
| | | |
| CCM (valeurs des Rf)^{a} | | |
| | (I) | 0.30 |
| | (II) | 0.62 |
| | (III) | 0.78 |
| | | |
| HPLC (Rt)^{b} | | 22.24 min |

| | | |
|---|---|---|
| ^{a}CCM sur gel de silice (MerckNo 5715). (I): EtOAc-MeOH (100:15). (II): *n*-BuOH-CH₃COOH-H₂O (3:1:1). (III): MeOH-CH₂Cl₂ (4:1). ^{b}Conditions d'HPLC: Uptisphere C₁₈ UP5ODB (250x7.8 mm, d.i.), Phase mobile: isocratique à 63% MeOH in H₂O, Débit: 1.5 ml/min, Détection: UV (220 nm). | | |

**Tableau 10. Assignements des données RMN du ¹H et du ¹³C du produit G dans du DMSO-d6 à 298 K.**

| **Position** | δ_{H} | δ_{C} |
|---|---|---|
| 1 | 7.97 (dd) | 120.9 |
| 2 | 7.87 (dd) | 137.0 |
| 3 | 7.72 (dd) | 124.2 |
| 4 | | 157.8 |
| 4a | | 121.2 |
| 5 | | nd |
| 5a | | 111.1 |
| 6 | | nd |
| 6a | | 139,6 |
| 7 | 5.08 (dd) | 63.7 |
| | 2.12 (dd) / 1.74 | |
| 8 | (dd) | 44.3 |
| 9 | | 68.1 |
| 10 | 2.73 (br s) | 38.3 |
| 10a | | 136,6 |
| 11 | | 155.4 |
| 11a | | 111.4 |
| 12 | | 186.2 |
| 12a | | 135,5 |
| OH-6 | 14.20 (br s)^{a} | |
| OH-7 | 4.89 (s) | |
| OH-9 | 4.60 (s) | |
| Me-9 | 1.31 (s) | 30.7 |
| OH-11 | 13.30 (br s)^{a} | |
| 1' | 5.65 (br s) | 99.5 |
| 2' | 4.02 (m) | 70,6 |
| 3' | 4.02 (m) | 71.0 |
| 4' | 3.36 (m) | 72.5 |
| 5' | 3.55 (dq) | 70.9 |
| OH-2' | 5.15 (d) | |
| OH-3' | 4.86 (d) | |
| OH-4' | 4.96 (d) | |
| Me-5' | 1.10 (d) | 19.8 |

| | | |
|---|---|---|
| ^{a}Signaux pouvant être interchangeables. | | |

**Tableau 11: Assignements des données RMN du ¹H et du ¹³C du produit P8 (aldgamycine H) dans du CD₃OD et dans du DMSO-D₈ à 298K.**

| **Position** | **PS (aldgamycin H)dans DMSO** | **P8 (aldgamycin H)dans CD3OD** | |
|---|---|---|---|
| | **dH (multi, J en Hz)** | **dH (multi, J en Hz)** | **dC** |
| 1 | | | 166.4 |
| 2 | 6.09 d.(15.3) | 6.03 d (15.4) | 120.9 |
| 3 | 6.56 dd (15.3 ; 10.6) | 6.64 dd (15.4 ; 10.6) | 152.1 |
| 4 | 2.72 ddq (10.6 ; 10.2 ; 6.8) | 2.83 ddq (10.6 ; 10.2 ; 6.7) | 41.9 |
| 5 | 3.27 br d (10.2) | 3.38 br d (10.2) | 86.8 |
| 6 | 0.98 br m 1.40 ddd(14.4, 12.0, 4.5) / 1.77 | 1.14 br m 1.59 ddd(14.0, 12.0, 4.1) / 1.81 | 34.6 |
| 7 | ddd(14.4, 11.0, 3.4) | ddd(14.0, 11.6, 3.1) | 32.0 |
| 8 | 2.41 ddq (12.0 ; 5.0; 6.9) | 2.56 ddq (11.9 ; 4.4; 7.0) | 45.2 |
| 9 | | | 202.8 |
| 10 | 7.02 d (15.4) | 6.92 d (15.4) | 126.7 |
| 11 | 6.20 dd (15.4 ; 9.4) | 6.35 dd (15.4 ; 9.4) | 144.1 |
| 12 | 3.43 dd (9.4 ; 2.0) | 3.43 dd (9.4 ; 1.9) | 59.0 |
| 13 | 2.99 dd (9.3 ; 2.0) | 3.06 dd (9.3 ; 1.9) | 59.2 |
| 14 | 1.37 br m | 1.42 br m | 49.8 |
| 15 | 5.21 dq (10.8 ; 6.3) | 5.40 dq (10.9 ; 6.2) | 68.7 |
| 16 | 1.25 d (6.3) | 1.36 d (6.2) | 17.4 |
| 17 | 1.15 d (6.8) | 1.26 d (6.7) | 18.4 |
| 18 | 0.89 d (7.5) | 1.02 d (6.8) | 16.6 |
| 19 | 1.10 d (6.9) 3.59 dd (10.3 ; 2.8) / 3.94 dd | 1.18 d (7.0) 3.70 dd (10.1 ; 2.7) / 4.14 dd | 16.8 |
| 20 | (10.3, 2.9) | (10.1, 2.9) | 67.1 |
| 1' | 4.39 d (8.0) | 4.60 d (7.9) | 103.0 |
| 2' | 3.39 dd (8.0 ; 6.2) | 3.54 d (7.9) | 70.8 |
| 3' | 1.32 dd (12.3 ; 8.3) / 1.38 dd | 1.44 dd (11.8; 8.3) / 1.55 dd (11.8 | 75.4 |
| 4' | (12.3 ; 2.4) | ; 2.4) | 36.3 |
| 5' | 3.73 ddq (8.3 ; 2.4 ; 6.2) | 3.90 ddq (8.3 ; 2.4; 6.1) | 66.6 |
| 6' | 3.70 dq (6.3 ; 3.9) | 3.90 q (6.5) | 68..5 |
| OH-2' | 4.75 d (6.2) | nd | |
| OH-3' | 3.89 br s | nd | |
| Me-5' | 1.07 d (6.2) | 1.18 d (6.1) | 20.4 |
| OH-6' | 4.47 d (3.9) | nd | |
| Me-6' | 0.98 d (6.3) | 1.17 d (6.5) | 15.7 |
| 1" | 4.49 d (8.1) | 4.60 d (8.0) | 101.2 |
| 2" | 3.02 dd (8.1 ; 2.7) | 3.11 dd (8.0 ; 2.9) | 81.8 |
| 3" | 3.66 dd (2.7 ; 2.6) | 3.80 dd (2.9 ; 2.8) | 80.5 |
| 4" | 3.09 ddd (9.6 ; 7.1 ; 2.6) | 3.20 ddd (9.5 ; 2.8) | 73.6 |
| 5" | 3.54 dq (9.6 ; 6.2) | 3.69 dq (9.5 ; 6.3) | 70.1 |
| OMe-2" | 3.41 s | 3.57 s | 58.5 |
| OMe-3" | 3.46 s | 3.60 s | 61.1 |
| OH-4" | 4.88 d (7.1) | nd | |
| Me-5" | 1.12 d (6.2) | 1.25 d (6.3) | 17.1 |

| | | | |
|---|---|---|---|
| nd : non détecté | | | |

**Tableau 12. Propriétés physicochimiques des produits P8 et P10a.**

| | | **P8** | **P10a** |
|---|---|---|---|
| Apparence | | Poudre incolore | Poudre incolore |
| Formule chimique | | C₃₆H₅₈O₁₄ | C₃₇H₅₆O₁₅ |
| | | | |
| Poids moléculaire | | 714 | 740 |
| | | | |
| Nano-ESI-MS (*m*/*z*) | | | |
| | Mode positif | 737.4 [M+Na]⁺, 563.3, 545.2, 389.2, 371.1, 289.1 | 763.44 [M+Na]⁺, 589.27, 563, 571.24, 559.19, 545.29, 417.16, 389.16, 371.16, 289.02 |
| | | | |
| UV λₘₐₓ nm dans MeOH | | 216.73, épaulements à 245.71 et 280 | 216.73, épaulement 245.71 et 278.09 |
| | | | |
| IR νₘₐₓ Cellule de diamant (cm⁻¹) | | 3431,2970,2931,2880, 1712, 1689, 1654, 1620, 1581,1454,1416,1382, 1354, 1326, 1279, 1263, 1236, 1172, 1159, 1117, 1082 | 3361,2968,2920,2851, 1799, 1712, 1653, 1623, 1593, 1562, 1508, 1457, 1417, 1383, 1356, 1324, 1282, 1238, 1194, 1173, 1159, 1117, 1084, 1047 |
| | | | |
| Solubilité relative | | | |
| | Très soluble | MeOH, EtOH, Me₂CO, *n*-BuOH, *1*-PrOH, 2-PrOH, CH₂Cl₂, EtOAc, CHCl₃ | MeOH, EtOH, Me₂CO, *n*-BuOH, *1*-PrOH, *2*-PrOH, CH₂Cl₂, EtOAc, CHCl₃ |
| | | | |
| | Insoluble | *n*-hexane, toluène, H₂O | *n*-hexane, toluène, H₂O |
| | | | |
| CCM (valeur du Rf)^{a} | | | |
| | (I) | 0.63 | 0.68 |
| | (II) | 0.71 | 0.74 |
| | (III) | 0.86 | 0.88 |
| | | | |
| HPLC (Rt)^{b} | | 23.21 min | 28.38 min |

| | | | |
|---|---|---|---|
| ^{a}CCM sur gel de silice (Merck No 5715). (I): EtOAc-MeOH (100:15). (II): *n-*BuOH-CH₃COOH-H₂O (3:1:1). (III): MeOH-CH₂Cl₂ (4:1). ^{b}Conditions d'HPLC: Uptisphère C₁₈ UP5ODB (250x7.8 mm, d.i.), Phase mobile: isocratique à 63% MeOH in H₂O, Débit: 1.5 ml/min, Détection: UV (220 nm). | | | |

**Tableau 13. Assignements des données RMN du ¹H et du ¹³C du produit P10a (aldgamycin G) dans du CD₃OD à 298K.**

| **Position** | **P10a (aldgamycin G)** | |
|---|---|---|
| | **δH (multi, J dans Hz)** | **δC** |
| 1 | | 166.3 |
| 2 | 6.03 d (15.5) | 121.0 |
| 3 | 6.64 dd (15.5 ; 10.7) | 151.8 |
| 4 | 2.84 ddq (10.7 ; 10.2; 6.8) | 41.8 |
| 5 | 3.42 d (10.2) | 86.8 |
| 6 | 1.16 m | 34.5 |
| 7 | 1.56 m / 1.82 ddd(14.0, 11.8, 2.9) | 31.9 |
| 8 | 2.54 ddq (11.8 ; 4.8; 7.0) | 45.2 |
| 9 | | 202.7 |
| 10 | 6.92 d (15.4) | 126.6 |
| 11 | 6.35 dd (15.4 ; 9.4) | 144.2 |
| 12 | 3.43.dd (9.4 ; 2.1) | 59.0 |
| 13 | 3.06 dd (9.3 ; 2.1) | 59.2 |
| 14 | 1.43 dm (10.8) | 49.8 |
| 15 | 5.40dq(10.8;6.3) | 68.7 |
| 16 | 1.36 d (6.3) | 17.4 |
| 17 | 1.24 d (6.8) | 18.2 |
| 18 | 1.01 d (6.8) | 16.6 |
| 19 | 1.18 d (7.0) | 16.8 |
| 20 | 3.70 dd(10.2 ; 3.1) / 4.13 dd(10.2 , 2.9) | 67.1 |
| 1' | 4.59 d (7.7) | 102.4 |
| 2' | 3.47 d (7.7) | 70.6 |
| 3' | | 86.6 |
| 4' | 1.61 dd(14.6 ; 11.1) / 1.92 dd(14.6 ; 2.1) | 41.1 |
| 5' | 3.84 ddq (11.1 ; 6.1 ; 2.1) | 67.0 |
| 6' | 4.50 q (6.5) | 82.2 |
| 7' | | 155.6 |
| OH-2' | nd | |
| Me-5' | 1.23 d (6.1) | 19.9 |
| Me-6' | 1.57 d (6.5) | 12.6 |
| 1" | 4.60 d (8.0) | 101.2 |
| 2" | 3.11 dd (8.0; 2.8) | 81.8 |
| 3" | 3.80 dd (2.8 ; 2.7) | 80.5 |
| 4" | 3.20 dd (9.6 ; 2.7) | 73.6 |
| 5" | 3.69 dq (9.6 ; 6.2) | 70.1 |
| OMe-2" | 3.57 s | 58.5 |
| OMe-3" | 3.60 s | 61.1 |
| OH-4" | nd | |
| Me-5" | 1.25 d (6.2) | 17.1 |

| | | |
|---|---|---|
| nd : non détecté | | |

**Tableau 14. Assignements des données RMN du ¹H et du ¹³C du produit P10b (swalpamycine B) dans du CD₃OD à 298K.**

| **Position** | **P10b (swalpamycine B)** | |
|---|---|---|
| | **δH (multi, J dans Hz)** | **δC** |
| 1 | | 166.7 |
| 2 | 5.88 d (15.4) | 121.4 |
| 3 | 6.62 dd (15.4 ; 10.0) | 152.5 |
| 4 | 2.79 m | 41.3 |
| 5 | 3.36 br d (10.1) | 87.3 |
| 6 | 1.21 m | 34.5 |
| 7 | 1.59 m^{a} / 1.70 ddd(14.4, 11.7, 3.2) | 31.9 |
| 8 | 2.53 m | 45.3 |
| 9 | | 205.7 |
| 10 | 6.46 d (15.0) | 123.9 |
| 11 | 7.08 dd (15.0; 10.9) | 142.4 |
| 12 | 6.28 dd (15.2 ; 10.9) | 133.4 |
| 13 | 6.08 dd (15.2 ; 9.3) | 142.0 |
| 14 | 2.46 m | 51.5 |
| 15 | 5.12 dq (10.1 ; 6.3) | 69.7 |
| 16 | 1.39 d (6.8) | 17.6 |
| 17 | 1.22 d (6.3) | 19.1 |
| 18 | 1.01 d (6.8) | 16.9 |
| 19 | 1.18 d (7.0) | 17.0 |
| 20 | 3.66 dd(9.9 ; 3.6) / 4.01 dd(9.9 , 3.4) | 68.5 |
| 1' | 4.56 d (8.0) | 102.9 |
| 2' | 3.54 d (8.0) | 70.8 |
| 3' | | 75.4 |
| 4' | 1.46 m / 1.54 m | 36.3 |
| 5' | 3.87 ddq (8.3 ; 6.2 ; 2.4) | 66.6 |
| 6' | 3.90 q (6.6) | 68.5 |
| OH-2' | nd | |
| OH-3' | nd | |
| Me-5' | 1.19 d (6.2) | 20.4 |
| OH-6' | nd | |
| Me-6' | 1.16 d (6.6) | 15.6 |
| 1" | 4.61 d (8.0) | 101.3 |
| 2" | 3.08 dd (8.0 ; 2.9) | 81.8 |
| 3" | 3.77 dd (2.9 ; 2.8) | 80.6 |
| 4" | 3.19 dd (9.6 ; 2.9) | 73.6 |
| 5" | 3.67 dq (9.6 ; 6.3) | 70.0 |
| OMe-2" | 3.53 s | 58.6 |
| OMe-3" | 3.59 s | 61.1 |
| OH-4" | nd | |
| Me-5" | 1.24 d (6.3) | 17.1 |

| | | |
|---|---|---|
| nd : non détecté | | |

## Revendications

1. Souche actinomycète *Saccharothrix* SA 103 déposée à la CNCM le 16 février 2004 sous le numéro I-3160 ou une souche mutante de celle-ci.

2. Procédé de production d'un bouillon de culture à partir d'une culture de la souche actinomycète *Saccharothrix* SA 103 et/ou d'au moins l'une de ses souches mutantes selon la revendication 1, **caractérisé en ce qu'**il comprend les étapes suivantes :
a) fermentation de ladite souche dans un bouillon nutritionnel afin d'obtenir le bouillon de culture ;
b) optionnellement, séparation du bouillon de culture obtenu à l'étape a).

3. Procédé de production d'un bouillon de culture selon la revendication 2, **caractérisé en ce que** la sépararation réalisée à l'étape b) optionnelle est une centrifugation et/ou une filtration et/ou une pasteurisation.

4. Bouillon de culture susceptible d'être obtenu par le procédé selon la revendication 2 ou 3.

5. Procédé de production d'un concentrat actif à partir du bouillon de culture selon la revendication 4, **caractérisé en ce qu'**il comprend les étapes suivantes :
a) extraction organique du bouillon de culture avec un solvant organique ;
b) optionnellement, déshydratation de la phase organique obtenue et/ou séchage *in vacuo* ;
c) optionnellement, mise en suspension du concentrat actif, de préférence filtration de la suspension obtenue, et répétition des étapes a) et b) d'extraction organique et de déshydratation.

6. Concentrat actif susceptible d'être obtenu par le procédé de la revendication 5.

7. Procédé de production d'un composé actif à partir du concentrat actif selon la revendication 6 par chromatographie liquide haute performance en phase inverse (reverse phase HPLC), de préférence précédée d'une chromatographie sur couche mince et/ou d'une chromatographie liquide à basse pression.

8. Procédé de production d'un composé actif selon la revendication 7, **caractérisé en ce que** le composé actif est une mutactimycine telle que la mutactimycine P11, la mutactimycine PR, la mutactimycine G ou la mutactimycine F, ou une aldgamycine telle que l'aldgamycine G, l'aldgamycine H ou l'aldgamycine P10b, ou les sels d'addition pharmaceutiquement acceptables, isomères, énantiomères, diastéréoismères, ainsi que les mélanges de ces composés actifs.

## Claims

1. Actinomycete strain *Saccharothrix* SA registered at CNCM on 16 February 2004 under the number I-3160 or a mutant strain thereof.

2. Method for producing a culture medium from a culture of the actinomycete strain *Saccharothrix* SA 103 and/or at least one of its mutant strains according to claim 1, **characterized in that** it comprises the following steps:
a) fermentation of said strain in a nutrient medium to obtain the culture medium;
b) optionally, separation of the culture medium obtained in step a).

3. Method for producing a culture medium according to claim 2, **characterized in that** the separation carried out in optional step b) is a centrifugation and/or a filtration and/or a pasteurization.

4. Culture medium capable of being obtained by the method according to either claim 2 or claim 3.

5. Method for producing an active concentrate from the culture medium according to claim 4, **characterized in that** it comprises the following steps:
a) organic extraction of the culture medium with an organic solvent;
b) optionally, dehydration of the organic phase and/or drying *in vacuo*;
c) optionally, placing of the active concentrate in suspension, preferably filtration of the suspension obtained, and repetition of the steps a) and b) of organic extraction and dehydration.

6. Active concentrate capable of being obtained by the method of claim 5.

7. Method for producing an active compound from the active concentrate according to claim 6 by reverse phase high performance liquid chromatography (reverse phase HPLC), preferably preceded by thin layer chromatography and/or low pressure liquid chromatography.

8. Method for producing an active compound according to claim 7, **characterized in that** the active compound is a mutactimycin such as mutactimycin P11, mutactimycin PR, mutactimycin G or mutactimycin F, or an aldgamycin such as aldgamycin G, aldgamycin H or aldgamycin P10b, or the pharmaceutically acceptable addition salts, isomers, enantiomers, diastereoisomers, and mixtures of these active compounds.

## Patentansprüche

1. Aktinomyceten-Saccharothrixstamm SA 103, der bei der CNCM am 16. Februar 2004 unter der Nummer I-3160 hinterlegt wurde, oder ein Mutantenstamm desselben.

2. Verfahren zur Herstellung einer Kulturlösung aus einer Kultur des Aktinomyceten-Saccharothrixstamms SA 103 und/oder mindestens einem seiner Mutantenstämme nach Anspruch 1, **dadurch gekennzeichnet, dass** es die folgenden Schritte umfasst:
a) Fermentierung des Stamms in einer Nährlösung, um die Kulturlösung zu herstellen,
b) optional Trennung der in Schritt a) hergestellten Kulturlösung.

3. Verfahren zur Herstellung einer Kulturlösung nach Anspruch 2, **dadurch gekennzeichnet, dass** die beim optionalen Schritt b) durchgeführte Trennung eine Zentrifugierung und/oder eine Filtration und/oder eine Pasteurisierung ist.

4. Kulturlösung, die mit dem Verfahren nach Anspruch 2 oder 3 herstellbar ist.

5. Verfahren zur Herstellung eines aktiven Konzentrats aus einer Kulturlösung nach Anspruch 4, **dadurch gekennzeichnet, dass** es die folgenden Schritte umfasst:
a) organische Extraktion der Kulturlösung mit einem organischen Lösungsmittel,
b) optional Dehydratation der hergestellten organischen Phase und/oder *in vacuo*-Trocknung,
c) optional Mischung des aktiven Konzentrats, vorzugsweise Filtration der hergestellten Mischung, und Wiederholung der Schritte a) und b) der organischen Extraktion und Dehydratation.

6. Aktives Konzentrat, das mit dem Verfahren nach Anspruch 5 herstellbar ist.

7. Verfahren zur Herstellung einer aktiven Verbindung aus einem aktiven Konzentrat nach Anspruch 6 durch Umkehrphasen-Hochleistungsflüssigkeitschromatographie (reverse phase HPLC), der vorzugsweise eine Dünnschichtchromatographie und/oder Niederdruck-Flüssigkeitschromatographie vorausgeht.

8. Verfahren zur Herstellung einer aktiven Verbindung nach Anspruch 7, **dadurch gekennzeichnet, dass** die aktive Verbindung ein Mutactimycin wie das Mutactimycin P11, das Mutactimycin PR, das Mutactimycin G oder das Mutactimycin F ist, oder ein Aldgamycin wie das Aldgamycin G, das Aldgamycin H oder das Aldgamycin PlOb, oder die pharmazeutisch akzeptablen Additionssalze, Isomere, Enantiomere, Diastereoismere und die Gemische dieser aktiven Verbindungen.
